# DEMANDE DE BREVET EUROPEEN

(11) **EP 4 649 982 A1**
(43) Date de publication de la demande: **19.11.2025**
(21) Numéro de dépôt: 25172163.5
(22) Date de dépôt: 24.04.2025
(51) Int. Cl.: A61M 16/00, A61M 16/12, A61M 16/20

(54) **APPAREIL DE FOURNITURE DE NO ADAPTÉ AU TRANSPORT TERRESTRE OU AÉRIEN**

(30) Priorité: 13.05.2024 FR 2404880
(71) Demandeur: INOSYSTEMS, 92160 Antony (FR)
(72) Inventeur: MARCHAL, Frederic, 92160 Antony (FR); BOULANGER, Thierry, 92160 Antony (FR); ARAMA, Bogdan, 92160 Antony (FR); SCHMITT, Mary, 92160 Antony (FR); BLANDIN, Yann, 92160 Antony (FR)
(74) Mandataire: Air Liquide

(57) **Abrégé**

L'invention concerne un appareil de délivrance (1) d'un gaz contenant du NO comprenant un circuit de gaz interne (200) avec des moyens de contrôle de débit (220, 230, 240) pour contrôler le flux de gaz, en particulier une électrovanne proportionnelle (220) et un dispositif de mesure débit (230). Des moyens de pilotage (210) coopèrent avec les moyens de contrôle de débit pour autoriser, interdire ou ajuster le débit gazeux au sein du circuit de gaz interne. Le dispositif de mesure débit comprend un capteur de débit bidirectionnel configuré pour opérer des mesures de débit positives et négatives du flux gazeux contenant du NO circulant dans le circuit de gaz interne, et fournir lesdites mesures de débit aux moyens de pilotage.

## Description

L'invention concerne un appareil de délivrance de NO bien adapté au transport terrestre ou aérien, et une installation de fourniture d'un mélange gazeux à base de NO à un patient, typiquement un mélange NO/azote (N₂), comprenant un tel appareil de délivrance de NO et un ventilateur médical délivrant un gaz respiratoire à base d'oxygène (i.e. ≥ 20% environ).

Le monoxyde d'azote inhalé (i.e. NO ou NOi) est un médicament gazeux couramment utilisé pour traiter des patients souffrant d'hypertension artérielle pulmonaire aiguë, en particulier les vasoconstrictions pulmonaires chez l'adulte ou l'enfant, y compris les nouveau-nés (PPHN), comme décrit par exemple par EP-A-560928 ou EP-A-1516639.

Pour mettre en œuvre une thérapie par NO inhalé, on utilise une installation de fourniture de gaz, aussi appelée installation d'administration de NO, comprenant un appareil de délivrance de NO et un ventilateur médical, c'est-à-dire un appareil d'assistance respiratoire, alimentant un circuit patient qui comprend généralement un ou plusieurs conduits flexibles reliés fluidiquement à une interface respiratoire, telle une sonde d'intubation trachéale ou analogue, servant à délivrer au patient à traiter, un mélange gazeux final contenant le NO.

Une telle installation de fourniture de gaz est décrite par exemple par EP3821929. Ce type d'installation est utilisé en milieu hospitalier pour administrer le traitement par NO et ainsi soigner les patients ayant besoin d'inhaler du NO pour traiter leur hypertension artérielle pulmonaire. Des installations de ce type sont également décrites par EP3233171, EP3410927, EP3410927, EP4209243, EP4241817, EP4241812 et EP4295882.

Dans une telle installation, l'appareil de délivrance de NO permet d'injecter un flux gazeux à base de NO, typiquement un mélange gazeux NO/azote, dans le circuit patient alimenté par ailleurs en un flux de gaz respiratoire contenant de l'oxygène (au moins environ 20% vol.), tel de l'air ou un mélange oxygène/azote (O₂/N₂), fourni par le ventilateur médical, de manière à obtenir un flux combiné, aussi appelé « mélange gazeux final », comprenant le NO à la posologie souhaitée, typiquement moins de 80 ppmv de NO, au moins environ 20% vol. d'oxygène et de l'azote (N₂), voire des impuretés inévitables.

Des moyens de contrôle de débit de l'appareil permettent de contrôler ou ajuster le débit de gaz contenant du NO, e.g. de mélange NO/N₂, dans le but d'obtenir le mélange gazeux combiné désiré, i.e. le mélange final contenant le NO à la posologie désirée.

De tels moyens de contrôle comprennent généralement un ou des actionneurs de type électrovanne proportionnelle et un capteur de débit, qui sont pilotés par un contrôleur électronique ou analogue, c'est-à-dire des moyens de pilotage, de manière à permettre de générer différentes valeurs de débits de NO en fonction de la concentration réglée par le médecin, i.e. la posologie souhaitée, et de la concentration en NO du gaz fourni par la ou les bouteilles de mélange NO/N₂.

Le NO doit pouvoir être fourni à un patient en ayant besoin en toutes circonstances, i.e. que le patient soit statique, par exemple alité au sein d'un établissement hospitalier, ou en mouvement, c'est-à-dire durant un transport éventuel dudit patient que ce transport ait lieu au sein même de l'établissement hospitalier, par exemple sur un brancard, un lit mobile ou analogue, ou dans un véhicule de transport terrestre ou aérien, comme dans une ambulance, un véhicule du SAMU ou autre, ou dans un aéronef, tel un avion ou un hélicoptère.

Dans tous les cas, il est nécessaire de pouvoir réguler et donc délivrer des débits sur une large plage de débit, typiquement entre 0 et 10 L/min, pour couvrir des posologies allant de 1 à 80 ppm de NO, en particulier pour une concentration en NO du gaz fourni par la ou les bouteilles qui soit inférieure à 1500 ppmv et habituellement comprise entre 200 et 1000 ppmv.

De ce fait, pour opérer une régulation précise du débit de NO entre quelques mL/min et 10 l/min environ, il est nécessaire d'utiliser un ou des capteurs de débit de NO très précis, c'est-à-dire typiquement d'une précision ou résolution inférieure à 1 mL/min.

Cependant, plus le capteur de débit est sensible, plus il va aussi capter les perturbations environnementales, en particuliers les chocs et/ou les vibrations, susceptibles de se produire durant un transport du patient au sein de l'établissement hospitalier et de façon encore plus notable, lorsqu'il se trouve dans un véhicule de transport terrestre ou aérien.

Or, ces perturbations environnementales, i.e. chocs et/ou vibrations, peuvent entraîner une saturation du signal en "faussant" la valeur moyenne du signal, ce qui se répercute négativement sur la régulation du débit de NO avec risque de fermeture intempestive de l'électrovanne proportionnelle fournissant le débit de gaz à base de NO, donc d'arrêter prématurément la délivrance de NO aux patients en engendrant alors un effet rebond négatif chez certains patients. Or, un tel effet rebond est évidemment indésirable pour les patients pour des raisons de sécurité sanitaire évidentes.

On connait WO2015168517 qui enseigne une installation de fourniture de mélange NO/air incluant un appareil de fourniture de NO/N₂ comprenant un conduit véhiculant le mélange NO/N₂, sur lequel sont agencés une valve et un capteur de débit. La valve peut être de différents types, à savoir valve proportionnelle, tout ou rien ou autre. Le capteur de débit est unidirectionnel. Il sert à mesurer le débit de mélange NO/N₂ uniquement dans le sens positif, c'est-à-dire uniquement en direction de l'injecteur agencé sur le circuit patient alimenté par le ventilateur médical. Un second capteur qui est de type bidirectionnel, est agencé dans l'injecteur lui-même, donc hors de l'appareil, et sert à suivre les flux positif et négatif provenant du ventilateur qui délivre un flux d'air oscillant.

Par ailleurs, US2023270960 enseigne une installation analogue à celle de WO2015168517, incluant un capteur bidirectionnel agencé sur la ligne d'air/O₂ pour détecter un éventuel flux négatif du flux d'air/O2 et, le cas échéant, de déclencher une alarme.

Aucun de ces documents ne permet de résoudre les problèmes de mesure causés par les perturbations environnementales, telles les vibrations, se produisant lors d'un transport de l'appareil de délivrance de NO, et de mauvaise régulation de la fourniture de NO, i.e. typiquement d'un mélange gazeux NO/N₂, qui en résultent.

Un problème est dès lors d'améliorer la sécurité du patient en proposant un appareil de délivrance de NO amélioré qui soit moins sensible aux perturbations environnementales, i.e. chocs et/ou vibrations, susceptibles d'entraîner une saturation du signal pendant un transport du patient au sein d'un établissement hospitalier ou lorsqu'il se trouve dans un véhicule de transport terrestre ou aérien.

Une solution concerne un appareil de délivrance, i.e. de fourniture, d'un gaz contenant du NO, tel un mélange gazeux NO/N₂, comprenant : un circuit de gaz interne pour acheminer un flux de gaz contenant du NO, comprenant des moyens de contrôle de débit configurés pour contrôler le flux de gaz contenant du NO au sein du circuit de gaz, lesdits moyens de contrôle de débit comprenant une électrovanne proportionnelle et un dispositif de mesure débit, et des moyens de pilotage coopérant avec les moyens de contrôle de débit pour autoriser, interdire ou ajuster le débit gazeux au sein dudit circuit de gaz.

De plus, selon l'invention, le dispositif de mesure débit comprend un capteur de débit bidirectionnel configuré pour opérer des mesures de débit positives et négatives du flux gazeux contenant du NO circulant dans le circuit de gaz, et fournir lesdites mesures de débit aux moyens de pilotage, lesdits moyens de pilotage traitant les mesures de débit positives et négatives provenant du dispositif de mesure débit pour piloter l'électrovanne proportionnelle

Selon le mode de réalisation considéré, l'appareil de l'invention peut comprendre l'une ou plusieurs des caractéristiques suivantes :
- les moyens de pilotage sont configurés pour piloter l'électrovanne proportionnelle à partir d'une valeur moyenne de débit prenant en compte les débits négatifs fournis par le capteur de débit bidirectionnel.
- le dispositif de mesure débit est agencé dans le circuit de gaz en amont de l'électrovanne proportionnelle, en considérant le sens de circulation du gaz.
- les moyens de pilotage comprennent au moins un microprocesseur.
- l'électrovanne proportionnelle et le dispositif de mesure débit forment tout ou partie d'un contrôleur de débit massique (MFC)

De plus, selon le mode de réalisation considéré, l'appareil de l'invention peut comprendre l'une ou plusieurs des caractéristiques additionnelles suivantes :
- il comprend des moyens de réglage de dose de NO configurés pour permettre à un utilisateur de régler une dose de NO désirée, i.e. une posologie.
- les moyens de réglage de dose de NO sont configurés pour permettre à un utilisateur de régler une dose de NO comprise entre 1 et 80 ppmv, en général une consigne de teneur en NO comprise entre 1 et 50 ppmv, typiquement entre 5 et 40 ppmv ou une autre dose.
- les moyens de pilotage coopèrent avec les moyens de réglage de dose de NO.
- la dose de NO réglée est fournie aux moyens de pilotage
- il comprend un afficheur graphique ou tout autre dispositif d'affichage, tel un écran tactile.
- les moyens de pilotage sont configurés pour commander un affichage sur l'afficheur graphique de la dose de NO réglée.
- les moyens de pilotage sont configurés pour commander un affichage sur l'afficheur graphique de seuils d'alarme NO haute et bas déterminés par les moyens de pilotage en fonction de la dose de NO réglée.
- le circuit de gaz interne de l'appareil véhiculant le gaz contenant le NO, tel un mélange NO/N₂, est raccordé fluidiquement à un circuit respiratoire véhiculant un gaz respiratoire contenant de l'oxygène, tel de l'air ou un mélange gazeux O₂/N₂ (>20% O₂), pour y injecter le gaz contenant le NO et obtenir un mélange gazeux combiné contenant du NO et de l'oxygène, c'est-à-dire le mélange final à administrer au patient.
- il comprend des moyens de mesure de concentration de NO pour déterminer la teneur en NO dans le mélange gazeux combiné contenant du NO et de l'oxygène, tel un capteur de NO, par exemple à cellule électrochimique.
- il comprend des moyens d'alarme, typiquement une alarme sonore et/ou visuelle.
- les moyens de pilotage sont configurés pour agir sur des moyens d'alarme pour déclencher une alarme, en particulier une alarme sonore et/ou visuelle.
- les moyens d'alarme peuvent comprendre un dispositif de type buzzer, et/ou un haut-parleur, ou tout autre dispositif permettant d'émettre un signal sonore audible par l'oreille humaine, i.e. par le personnel soignant.
- les moyens d'alarme peuvent comprendre un dispositif lumineux, par exemple comprenant une ou des diodes (LED) ou analogue.
- les moyens d'alarme peuvent comprendre un affichage d'un message d'alerte ou analogue sur l'afficheur graphique.
- les moyens de pilotage sont configurés pour commander un affichage sur l'afficheur graphique de la teneur en NO dans le mélange gazeux combiné ayant été déterminée par les moyens de mesure de concentration de NO.
- les moyens de réglage de dose sont configurés pour permettre à l'utilisateur de modifier la dose de NO (D_{NO}) et/ou les seuils d'alarme NO haut et bas.
- il est alimenté en un gaz contenant du NO en une proportion initiale donnée, (e.g. un mélange NO/N₂), en général une proportion initiale de NO comprise entre 100 et 1500 ppmv, typiquement entre 200 et 1000 ppmv.
- il est alimenté en un mélange gazeux formé d'azote et de NO.
- les moyens de réglage de dose de NO sont configurés pour permettre à l'utilisateur de régler une dose de NO, i.e. consigne de teneur en NO, correspondant à la proportion de NO souhaitée dans le mélange gazeux combiné, c'est-à-dire après mélange du mélange NO/N₂ avec le flux de gaz respiratoire à base d'oxygène, i.e. une posologie en NO.
- les moyens de réglage de dose de NO font partie d'une IHM (interface homme-machine) ou IGU (interface graphique utilisateur).
- l'IHM comprend l'afficheur graphique, de préférence à affichage en couleurs.
- les moyens de réglage de dose de NO comprennent une ou des touches virtuelles tactiles ou analogues, actionnables par l'utilisateur, s'affichant sur l'afficheur graphique de type écran digital à dalle tactile.
- il comprend des moyens de mémorisation.
- les moyens de mémorisation comprennent une mémoire informatique, telle une mémoire flash, une RAM ou analogue.
- les moyens de contrôle de débit sont agencés sur le circuit de gaz interne.
- le contrôleur de débit massique (Mass Flow Controler) ou MFC comprend au moins l'électrovanne proportionnelle pilotée par les moyens de pilotage et le capteur de débit.
- les moyens de pilotage comprennent un (ou des) (micro)processeur agencé sur une (ou des) carte électronique.
- les moyens de pilotage forment, sont ou comprennent un (micro)contrôleur ou analogue.
- les moyens de pilotage comprennent un (ou des) (micro)processeur mettant en œuvre un ou des algorithmes, notamment un (ou des) algorithme de pilotage des moyens de contrôle de débit, un (ou des) algorithme de traitement des mesures de débit et/ou un (ou des) algorithme de traitement des mesures de concentration en NO, NO₂ et/ou en O₂....
- les moyens de mémorisation sont agencés sur la carte électronique.

L'invention porte aussi sur une installation de fourniture d'un gaz contenant du NO comprenant l'appareil de délivrance de NO selon l'invention alimenté en un mélange gazeux NO/N₂ par une (ou des) source de mélange NO/N₂, et un ventilateur médical configuré pour fournir un flux de gaz respiratoire contenant de l'O₂.

Selon le mode de réalisation considéré, l'installation de fourniture d'un gaz contenant du NO de l'invention peut comprendre l'une ou plusieurs des caractéristiques suivantes :
- le ventilateur médical est configuré pour fournir un flux de gaz respiratoire contenant au moins 20%vol. environ d'O₂, typiquement un mélange NO/N₂ ou de l'air.
- l'appareil de délivrance de NO et le ventilateur médical sont raccordés fluidiquement à un circuit respiratoire pour lui fournir le flux de gaz contenant le NO (e.g. NO/N₂) et le flux de gaz contenant de l'oxygène (e.g. air ou O₂/N₂) et ainsi obtenir un mélange gazeux combiné à fournir au patient, par exemple via une sonde d'intubation trachéale ou autre.
- un capteur de débit est agencé dans le circuit respiratoire entre le ventilateur médical et le dispositif d'injection.
- un dispositif d'injection est agencé dans le circuit respiratoire, en aval du capteur de débit.
- le dispositif d'injection est configuré pour permettre d'injecter le gaz contenant du NO provenant de l'appareil de délivrance de NO dans le flux de gaz respiratoire contenant de l'O₂ provenant du ventilateur médical pour obtenir le mélange gazeux combiné à fournir au patient en ayant besoin.
- le dispositif d'injection est configuré pour opérer un mélange du gaz contenant du NO provenant de l'appareil de délivrance de NO avec le flux de gaz respiratoire contenant de l'O₂ acheminé par le circuit respiratoire, et obtenir un mélange gazeux combiné contenant du NO et de l'oxygène, voire d'autres composés comme de l'azote ou des impuretés inévitables.
- le dispositif d'injection comprend une première entrée de gaz alimentée en flux de gaz respiratoire contenant de l'O₂, i.e. provenant du ventilateur médical.
- le dispositif d'injection comprend en outre une seconde entrée de gaz alimentée en gaz contenant du NO provenant de l'appareil de délivrance de NO.
- le dispositif d'injection comprend en outre une sortie de gaz fournissant le mélange gazeux combiné contenant du NO et de l'oxygène, obtenu par mélange, au sein du dispositif d'injection, du gaz contenant du NO (e.g. mélange NO/N₂) avec le flux de gaz respiratoire contenant de l'O₂ (e.g. air ou mélange O₂/N₂).
- le ventilateur médical délivre de l'air ou un mélange oxygène/azote, i.e. en tant que gaz respiratoire contenant au moins 20% vol. environ d'oxygène, de préférence au moins 21% vol. environ d'oxygène.
- le ventilateur médical comprend une soufflante motorisée (i.e. turbine, compresseur ou analogue) délivrant le gaz respiratoire, typiquement de l'air ou un mélange oxygène/azote ou, selon un autre mode de réalisation, un circuit de gaz interne comprenant une ou des vannes proportionnelles pour acheminer le gaz et contrôler sa fourniture, notamment son débit. Un tel ventilateur est généralement alimenté en gaz respiratoire par une (ou des) prise murale alimentée en gaz par un réseau de canalisations d'un établissement ou bâtiment hospitalier, typiquement de l'air ou un mélange oxygène/azote.
- le ventilateur médical comprend des moyens ou un dispositif de commande, telle une (ou des) carte de commande électronique. De préférence, les moyens de commande du ventilateur médical pilotent ou commandent la soufflante motorisée ou, selon le cas, les valves proportionnelles du ventilateur médical.
- le ventilateur médical est de type HFO ou comprend une fonction de HFO, c'est-à-dire qu'il est apte à produire des oscillations à haute fréquence.
- la (les) source de NO alimentant l'appareil de délivrance de NO contient un mélange gazeux NO/N₂ contenant entre 100 et 2000 ppmv de NO, le reste étant de l'azote (N₂), de préférence entre 100 et 1000 ppmv de NO, de préférence conditionné à une pression comprise entre 10 et 250 bar abs, typiquement à plus de 100 bar abs (avant début de soutirage).
- la (ou les) source de NO est ou comprend une (ou des) bouteille de gaz, de préférence ayant une contenance comprise entre 0,5 et 50 L (équivalent en eau).
- la (les) bouteille de gaz comprend un corps cylindrique en acier ou en alliage d'aluminium et est équipée d'un robinet simple (sans détendeur) ou à détendeur intégré ou RDI, de préférence un RDI, protégé par un capotage de protection, par exemple en métal ou polymère.
- le circuit respiratoire (i.e. circuit patient) comprend une branche inspiratoire et une branche expiratoire, typiquement des conduites flexibles formant la branche inspiratoire et la branche expiratoire, par exemple des tuyaux en polymère.
- la branche inspiratoire et la branche expiratoire, e.g. des conduites flexibles, sont raccordées à une pièce de jonction, telle une pièce en Y.
- la branche inspiratoire et/ou la branche expiratoire sont reliées fluidiquement à une interface respiratoire patient, de préférence via la pièce de jonction.
- l'interface respiratoire patient comprend une sonde d'intubation trachéale ou un masque respiratoire, ou autre.
- le circuit respiratoire, en particulier la branche inspiratoire, comprend un humidificateur de gaz.
- l'humidificateur de gaz est agencé en aval du dispositif d'injection, par exemple un module d'injection de NO, de manière à pouvoir humidifier le gaz avant son administration par inhalation au patient.
- le ventilateur et l'appareil de délivrance de NO sont alimentés électriquement par une ou des sources de courant électrique, typiquement le secteur (110/220V) et/ou une ou des batteries rechargeables.

Selon un autre aspect, l'invention concerne aussi une méthode de traitement thérapeutique d'une personne, i.e. un patient humain (i.e. adulte, enfant, adolescent ou nouveau-né), souffrant d'hypertension pulmonaire et/ou d'hypoxie, engendrant des vasoconstrictions pulmonaires ou analogues, comprenant une administration par inhalation à la personne en ayant besoin, d'un mélange gazeux comprenant de 1 à 80 ppmv de NO, typiquement moins de 40 ppmv, et au moins 20 %vol. d'oxygène environ, de préférence au moins 21 %vol. d'oxygène environ, au moyen d'une installation de fourniture de gaz, telle celle décrite ci-avant selon l'invention, comprenant un appareil de délivrance de NO selon l'invention assurant une délivrance de NO, de manière à traiter (au moins partiellement) ladite hypertension pulmonaire et/ou ladite hypoxie, qui peut être causée par une (ou des) pathologie ou autres troubles pulmonaires typiquement de type PPHN (hypertension pulmonaire persistante du nouveau-né) ou SDRA (syndrome de détresse respiratoire aigüe), ou encore engendrée par une opération de chirurgie cardiaque avec mise du patient sous circulation sanguine extracorporelle (CEC).

### Définitions

D'une façon générale, dans le cadre de l'invention :
- « ppmv » signifie partie par million en volume,
- « %vol. » » signifie pourcentage en volume.
- « NO » désigne le monoxyde d'azote.
- « NO₂ » désigne le dioxyde d'azote.
- « N₂ » désigne l'azote.
- « O₂ » désigne l'oxygène.
- les termes « concentration », « quantité », « proportion », « dose » et « teneur » sont considérés comme équivalents.
- les termes « moyen de/à/pour » sont considérés comme totalement équivalents et substituables par les termes « dispositif de/à/pour », par exemple les termes « moyens de pilotage » peuvent être remplacés par « dispositif de pilotage », les termes « moyens à vannes » peuvent être remplacés par « dispositif à vannes », les « moyens de mémorisation» peuvent être remplacés par « dispositif de mémorisation» ...
- par « mesure de débit », on entend une valeur de débit (e.g. une valeur numérique) ou un signal représentatif d'une telle valeur de débit reflétant ou correspondant à un débit gazeux mesuré par un capteur de débit, tel un capteur de débit massique.
- par « mesure de pression », on entend une valeur de pression (e.g. une valeur numérique) ou un signal représentatif d'une telle valeur de pression reflétant ou correspondant à la pression gazeuse mesurée par un capteur de pression.
- par « mesure de concentration », on entend une valeur de teneur en un composé gazeux donné, comme le NO, le NO₂ ou l'O₂, c'est-à-dire une valeur numérique ou un signal représentatif d'une telle valeur de teneur reflétant ou correspondant à la proportion (i.e. quantité) du composé gazeux considéré dans un mélange gazeux donné, mesurée par des moyens de mesure de concentration, telles des capteurs à cellules électrochimiques.

L'invention va maintenant être mieux comprise grâce à la description détaillée suivante, faite à titre illustratif mais non limitatif, en référence aux figures annexées parmi lesquelles :
Fig. 1 schématise un mode de réalisation d'une installation d'administration de gaz comprenant un appareil de délivrance de NO selon l'invention.
Fig. 2 schématise un mode de réalisation de l'architecture interne de l'appareil de délivrance de NO selon l'invention, tel celui de l'installation de Fig. 1.
Fig. 3 représente une courbe de débit obtenue avec un capteur unidirectionnel classique, en l'absence de toute vibration.
Fig. 4 représente une courbe de débit obtenue avec un capteur unidirectionnel classique, en présence de vibrations, comme celles se produisant pendant un transport du malade.
Fig. 5 représente une courbe de débit obtenue en présence de vibrations mais avec un capteur particulier conformément à la présente invention.

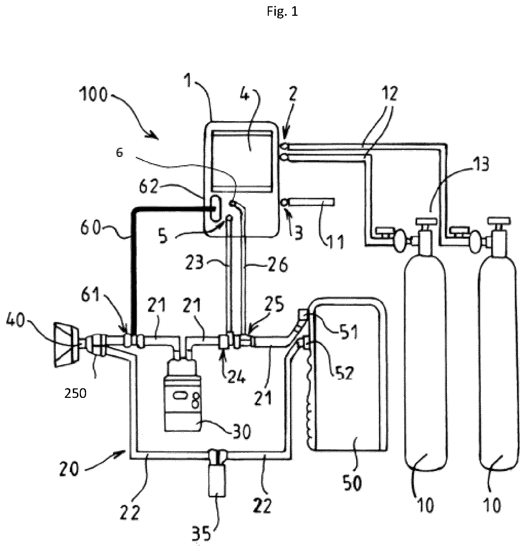

Fig. 1 schématise un mode de réalisation d'une installation d'administration de gaz 100 selon l'invention comprenant un appareil de délivrance de NO 1 selon l'invention permettant de fournir un mélange gazeux à base de monoxyde d'azote (NO), typiquement un mélange gazeux NO/N₂, et un ventilateur médical 50 fournissant un gaz contenant au moins 20%vol. d'oxygène, typiquement de l'air, un mélange gazeux O₂/N₂ ou autre.

L'installation 100 comprend ici deux bouteilles de gaz sous pression 10 contenant chacune un mélange gazeux à base de NO, à savoir un mélange gazeux NO/N₂ contenant ici entre 100 et 1500 ppmv de NO (reste N₂), par exemple 450 ou 800 ppm vol. de NO (reste N₂), ou toute autre concentration adéquate, qui alimentent en mélange NO/N₂, le dispositif ou appareil 1 de délivrance ou fourniture de NO permettant de suivre et contrôler la fourniture du mélange gazeux NO/N₂.

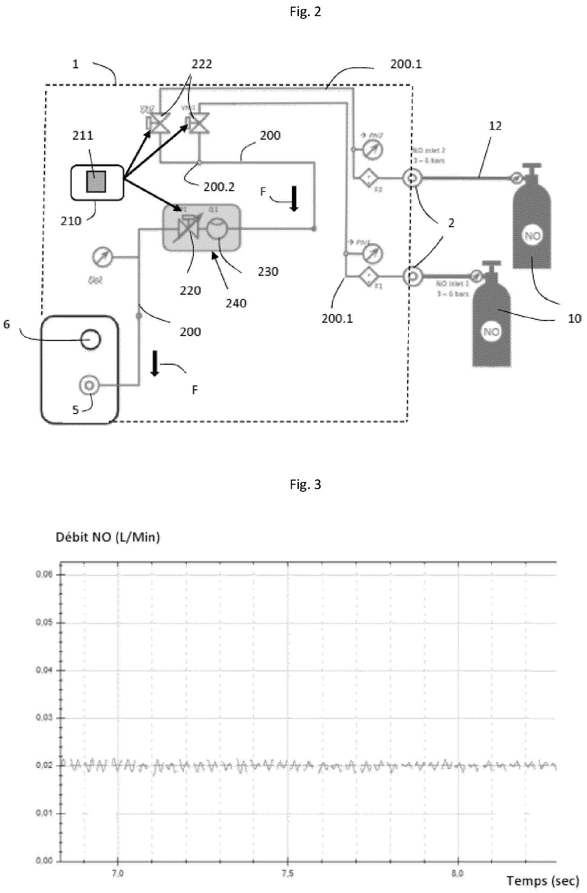

Les bouteilles de gaz 10 sont reliées fluidiquement à l'appareil 1 de fourniture de NO, via des lignes d'amenée de gaz 12, tel que des tuyaux ou conduits flexibles ou analogues, équipées de dispositifs de régulation et/ou de suivi de la pression de gaz, tels que détendeur de gaz 13, manomètres... Les lignes d'amenée de gaz 12 sont reliées à une ou plusieurs entrées de gaz 2 de l'appareil de délivrance de NO 1 qui alimentent un circuit de gaz interne 200, comme schématisé en Fig. 2, servant à acheminer le gaz au sein de l'appareil de fourniture de NO 1, c'est-à-dire dans le boitier ou carcasse de l'appareil 1 de délivrance de NO selon l'invention.

Dans le mode de réalisation de Fig. 2, le circuit de gaz interne 200 est relié à deux entrées de gaz 2 agencées en parallèle et alimentant chacune un tronçon d'entrée 200.1 dédié du circuit de gaz interne 200. Des vannes de contrôle 222 ou analogue pilotés par des moyens de pilotage 210 de l'appareil 1, contrôlent le passage du flux de NO/N₂ dans ces tronçons d'entrée 200.1 et dans le reste du circuit 200. Ces deux tronçons 200.1 se rejoignent en un site de raccordement 200.2 du circuit 200 qui est situé en aval des vannes de contrôle 222.

L'appareil de délivrance de NO 1 comprend par ailleurs une entrée d'oxygène 3 reliée fluidiquement, via une ligne d'amenée d'oxygène 11, tel un tuyau flexible ou analogue, à une source d'oxygène (non montrée), par exemple une bouteille d'oxygène sous pression ou un réseau hospitalier. Ceci permet d'alimenter le circuit de gaz interne 200 en oxygène quand cela est nécessaire.

Le ventilateur médical 50, c'est-à-dire un appareil d'assistance respiratoire, fournit un flux de gaz respiratoire à base d'oxygène, c'est-à-dire contenant au moins 20%vol. d'oxygène environ, préférentiellement au moins 21%vol. d'oxygène environ, tel de l'air ou un mélange oxygène/azote (N₂/O₂).

Le ventilateur médical 50 et l'appareil de fourniture de NO 1 de l'installation 100 sont en communication fluidique avec un circuit respiratoire 20, aussi appelé circuit patient, en particulier avec une ligne d'alimentation en gaz ou branche inspiratoire 21, qui sert à acheminer le flux gazeux vers l'interface respiratoire 40 fournissant le flux gazeux au patient, c'est-à-dire un mélange gazeux combiné, i.e. mélange final, contenant la posologie en NO souhaitée. Ce mélange gazeux combiné est obtenu par mélange du flux à base d'oxygène (e.g. air ou mélange O₂/N₂) provenant du ventilateur médical 50 et du flux contenant le NO, i.e. le mélange gazeux NO/N₂, délivré par l'appareil de délivrance de NO 1.

Pour ce faire, l'appareil de délivrance de NO 1 fournit ou injecte le mélange NO/N₂ dans le circuit respiratoire 20 véhiculant le flux à base d'oxygène, via un conduit ou une ligne d'injection 23, reliant fluidiquement le port de sortie 5 du circuit de gaz interne 200 de l'appareil de fourniture de NO 1 à un dispositif d'injection 24 agencé sur la ligne d'alimentation en gaz 21.

Le dispositif d'injection 24 est configuré pour opérer un mélange du gaz contenant du NO provenant de l'appareil de délivrance de NO 1 avec le flux de gaz respiratoire contenant de l'O₂ provenant du ventilateur 50 et acheminé par la branche inspiratoire 21 du circuit respiratoire 20, et obtenir un mélange gazeux combiné contenant du NO et de l'oxygène, c'est-à-dire le mélange gazeux final à administrer au patient contenant essentiellement du NO à la posologie désirée, de l'azote (N₂) et de l'oxygène (O₂), et éventuellement des impuretés inévitables (e.g. argon, CO₂, NO₂, ....), c'est-à-dire un mélange gazeux final NO/N₂/O₂.

La branche inspiratoire 21 comprend en outre un humidificateur de gaz 30 agencé en aval du dispositif d'injection 24, pour humidifier le flux de gaz final, e.g. le mélange gazeux combiné NO/N₂/O₂, avant qu'il ne soit administré par inhalation au patient à traiter, au moyen d'une interface respiratoire 40, telle une sonde d'intubation trachéale, un masque respiratoire ou analogue.

Une ligne de récupération des gaz expirés par le patient forme une branche expiratoire 22 du circuit patient 20. Elle est reliée fluidiquement à la branche inspiratoire 21 via une pièce de raccordement 250, telle une pièce en Y.

La branche inspiratoire 21 est, à son extrémité amont, raccordée fluidiquement à un port de sortie 51 du ventilateur médical 50, tel un connecteur, raccord ou analogue, de manière à récupérer et acheminer le gaz à base d'oxygène, typiquement de l'air ou mélange N₂/O₂ fourni par le ventilateur médical 50, alors que la branche expiratoire 22 véhiculant les gaz expirés est raccordée fluidiquement à un port d'entrée 52 du ventilateur médical 50, tel un connecteur, raccord ou analogue, de manière à retourner au ventilateur médical 50 tout ou partie du débit des gaz expirés par le patient.

Par ailleurs, un capteur de débit 25, par exemple du type à fil chaud ou à différentiel de pression, est agencé sur le circuit respiratoire 20, en particulier sur la branche inspiratoire 21, entre le ventilateur 50 et le dispositif d'injection 24. Le capteur de débit 25 est relié à un port de connexion 6 de l'appareil de délivrance de NO 1, via une (des) ligne de mesure de débit 26 venant se raccorder audit port de 6. Il sert à mesurer le débit de gaz délivré par le ventilateur 50, tel de l'air ou N₂/O₂, circulant dans la branche inspiratoire 21, en amont du dispositif d'injection.

Ces mesures de débit opérées par le capteur de débit 25 permettent de contrôler ou réguler plus efficacement la délivrance du flux de NO (i.e. N₂/O₂) par l'appareil de délivrance de NO 1, en particulier le débit de NO, puisque les mesures de débit opérées par le capteur de débit 25 sont retournées, via la ligne de mesure de débit 26 (i.e. câbles électriques ou analogues) et le port de connexion 6, à des moyens de pilotage 210 à (micro)processeur 211, typiquement un contrôleur, qui traitent ces mesures de débit.

Pendant le fonctionnement de l'appareil 1, le flux de NO/N₂ transite dans le circuit de gaz interne 200 dans le sens allant des entrées de gaz 2 en direction du port de sortie 5, c'est-à-dire du patient P (i.e. sens de flèche F en Fig. 2.

Par ailleurs, une électrovanne proportionnelle 220 pilotée par les moyens de pilotage 210, est agencée sur le circuit de gaz interne 200, et sert à contrôler ou ajuster le flux gazeux qui y circule en direction du port de sortie 5 et de la ligne d'injection 23, c'est-à-dire vers le dispositif d'injection 24, pendant le fonctionnement normal de l'appareil 1.

Plus précisément, l'électrovanne proportionnelle 220 est associée à un capteur de débit 230 de manière à former un contrôleur de débit massique 240 ou MFC (pour *Mass Flow Controller*).

L'électrovanne proportionnelle 220 et le capteur de débit 230 coopèrent avec les moyens de pilotage 210, c'est-à-dire un (ou des) dispositif de pilotage ou (micro)contrôleur. En particulier, le capteur de débit 230 mesure le débit de gaz contenant du NO circulant dans le circuit 200 et retourne les mesures aux moyens de pilotage 210 et ceux, en réponse à ces mesures de débit, contrôlent l'ouverture ou la fermeture de l'électrovanne proportionnelle 220 afin d'ajuster le débit de gaz traversant ladite électrovanne proportionnelle 220 en direction du port de sortie 5.

Comme expliqué ci-après, le capteur de débit 230 utilisé dans le cadre de l'invention est un capteur particulier, à savoir un capteur de débit bidirectionnel configuré pour permettre la mesure de débits positifs et négatifs, c'est-à-dire dans le sens et dans le sens contraire au flux normal (i.e. flux vers le patient).

Par ailleurs, les moyens de pilotage 210 comprennent une (des) carte électronique comprenant un (ou plusieurs) microprocesseur(s) 211 mettant en œuvre un ou des algorithmes.

Les moyens de pilotage 210 permettent notamment d'ajuster ou contrôler le débit de gaz à base de NO au sein du circuit 200, y compris dans les tronçons 200.1, en pilotant les vannes 222 et l'électrovanne proportionnelle 210, typiquement d'ouvrir ou fermer une ou des (électro)vannes, pour obtenir un débit de gaz à base de NO donné, c'est-à-dire autoriser ou stopper tout ou partie du débit de gaz.

Bien entendu, les moyens de pilotage 210 permettent en outre de traitement des informations, mesures ou autres, d'opérer des calculs et/ou de contrôler ou commander d'autres éléments électromécaniques de l'appareil 1, tels que les affichages ...

En particulier, les moyens de pilotage 210 peuvent déterminer le débit de NO à fournir pour obtenir la teneur en NO souhaitée dans le mélange combiné, c'est-à-dire la posologie en NO désirée, à partir notamment d'une consigne de teneur en NO réglée et/ou fixée par l'utilisateur, c'est-à-dire d'une dose de NO réglée par l'utilisateur, de la composition du mélange gazeux NO/N₂ fourni par les bouteilles de NO 10, en particulier de la teneur en NO dans ce mélange NO/N₂ qui peut être mémorisée par l'appareil 1, et des mesures de débit opérées par le capteur de débit 25 agencé sur la branche inspiratoire 21 et relié par une ligne de mesure de débit 26 aux moyens de pilotage 210, via le port de connexion 6 au capteur de débit 25.

Par ailleurs, le circuit de gaz 200 de l'appareil de fourniture de NO 1 peut aussi comprendre d'autres éléments ou composants, en particulier un (ou des) capteur de pression supplémentaire, un (ou des) capteur de débit additionnel ou débitmètre et/ou des dispositifs à orifice calibré ou autres (non montrés). Il peut aussi comprendre une ligne de secours (non montrée) utilisable par exemple en cas de dysfonctionnement de l'électrovanne 210, laquelle bipasse l'électrovanne 210.

L'appareil de délivrance de NO 1 comprend aussi une interface graphique utilisateur ou IGU comprenant un afficheur graphique 4, de préférence un écran tactile, c'est-à-dire à dalle tactile, servant à afficher différentes informations ou données, icones, courbes, alarmes..., ainsi que des moyens de sélection, telles des touches de sélection virtuelles et/ou des pavés ou fenêtres, servant notamment à opérer des choix, des sélections ou encore à entrer des informations, telles des valeurs désirées (e.g. débit, dosage de NO...), ou toute autre information ou donnée utile au personnel soignant. De préférence, l'affichage est en couleurs.

Toutefois, selon un autre mode de réalisation, l'appareil 1 peut aussi comprendre un ou des organes de sélection mécaniques, i.e. non-virtuels, comme un ou des boutons ou touches de sélection actionnable par pression digitale de l'utilisateur, un (ou des) bouton de sélection rotatif ou autre. Les sélections ou choix opérées via ce ou ces organes de sélection mécaniques peuvent s'afficher sur l'afficheur graphique 4 de l'IGU.

L'alimentation électrique de l'appareil de fourniture de NO 1, en particulier des composants nécessitant du courant électrique pour fonctionner, tels les moyens de pilotage 210, l'afficheur graphique 4, les capteurs..., est assurée classiquement par une source de courant électrique et/ou des moyens d'alimentation électrique (non montrés), par exemple une liaison au courant du secteur (110/220V) de type cordon électrique et prise de raccordement, et/ou une (ou des) batterie d'alimentation électrique, de préférence rechargeable, et/ou un transformateur de courant. L'alimentation électrique du ventilateur médical 50 est assurée de façon analogue, notamment par une liaison au courant du secteur ou une batterie interne.

En outre, l'installation 100 comprend aussi une ligne de prélèvement de gaz 60 qui relie fluidiquement la branche inspiratoire 21 à l'appareil de fourniture de NO 1.

La ligne de prélèvement de gaz 60 permet d'assurer un monitorage, c'est-à-dire un suivi, de la composition du mélange gazeux combiné contenant le NO, l'O₂ etc... afin de s'assurer que les teneurs en NO, en O₂ et aussi en espèces toxiques NO₂ pouvant se former sont conformes, en particulier que la proportion de NO est bien comprise entre des seuils d'alarme NO haut et bas fixés, c'est-à-dire entre des teneurs de NO maximale et minimale acceptables. Dans le cas contraire, les moyens de pilotage de l'appareil 1 déclenchent une alarme sonore et/ou une alarme visuelle afin d'alerter le personnel soignant.

La ligne de prélèvement de gaz 60, aussi appelée ligne de monitorage, vient se raccorder fluidiquement, en un site de raccordement 61 sur Fig. 1, à la ligne d'alimentation en gaz 21, entre l'humidificateur 30 et la pièce de jonction 250, i.e. pièce en Y, typiquement à proximité immédiate de la pièce de jonction 250, et par ailleurs à un port d'entrée 62 du dispositif de fourniture de NO 1, par exemple un port 62 porté par un connecteur, raccord ou analogue, permettant le raccordement de la ligne de prélèvement de gaz 60, tel un tuyau flexible ou analogue. Les échantillons de gaz prélevés sont analysés au sein d'une ligne de calibration comprenant des capteurs de NO₂, de NO et d'O₂, telles des cellules électrochimiques formant un analyseur de gaz interne (non montré) relié électriquement aux moyens de pilotage 210. En effet, pour des raisons de sécurité, il convient de vérifier que la composition du gaz final, i.e. mélange gazeux combiné, est conforme à celle du mélange gazeux NO/N₂/O₂ souhaité devant être administré au patient, notamment pour s'assurer qu'il ne contient pas de quantité excessive d'espèces NO₂ toxiques, que sa teneur en oxygène n'est pas hypoxique, et que sa teneur en NO correspond à la posologie souhaitée, i.e. la dose de NO à administrer par inhalation qui est choisie par le personnel soignant, i.e. médecin ou analogue.

La quantité de NO désirée présente dans le mélange combiné administré au patient, qui est typiquement comprise entre 1 et 80 ppmv, c'est-à-dire la valeur de consigne de NO ou posologie, peut être entrée et/ou ajustée et/ou modifiée par l'utilisateur, de préférence via l'IHM, grâce à des moyens de réglage de dose ou analogue, tels une ou des touches, boutons rotatifs, des curseurs ou autres, par exemple une ou des touches virtuelles affichées sur l'écran d'affichage 4. Par exemple, ils comprennent une touche « + » permettant d'incrémenter une valeur de dose de NO et une touche « - » permettant de décrémenter une valeur de dose de NO. Typiquement, l'incrémentation ou la décrémentation se fait de +/- 1 ppmv. De même, la teneur en NO dans le mélange gazeux NO/N₂ provenant des bouteilles 10 peut être ajustable ou non, c'est-à-dire mémorisée une fois pour toute ou réglable par l'utilisateur, au sein d'un menu dédié, par exemple en utilisant, là encore, les moyens de réglage.

Comme déjà expliqué, le NO doit pouvoir être fourni à un patient en ayant besoin en toutes circonstances, i.e. que le patient soit statique, par exemple alité au sein d'un établissement hospitalier, ou en mouvement, c'est-à-dire durant un transport éventuel dudit patient. Il est alors nécessaire de pouvoir réguler et délivrer des débits de NO sur une large plage de débit, typiquement entre 0 et 10 L/min, pour couvrir des posologies allant de 1 à 80 ppm de NO.

Ceci requiert d'utiliser un capteur de débit de NO 230 très précis, c'est-à-dire typiquement d'une précision ou résolution inférieure à 1 mL/min.

On utilise classiquement à cette fin un capteur de débit unidirectionnel dit « classique » qui est conçu pour ne mesurer le débit que dans un sens de circulation du gaz, à savoir en direction du patient (cf. flèche F en Fig. 2). Typiquement, un tel capteur de débit unidirectionnel mesure le débit gazeux en utilisant uniquement la partie positive du signal de débit.

Le capteur de débit 230 du MFC 240 peut être du type à différentiel de pression, massique ou autre, et coopère avec les moyens de pilotage 210 pour leur fournir des mesures de débit du flux de NO/N₂.

Or, plus le capteur de débit 230 est sensible, plus il va aussi capter les perturbations environnementales, en particuliers les chocs et/ou les vibrations, susceptibles de se produire durant un transport du patient au sein de l'établissement hospitalier et de façon encore plus notable, lorsqu'il se trouve dans un véhicule de transport terrestre ou aérien, tel une ambulance ou un hélicoptère.

Le problème est que ces perturbations environnementales, i.e. chocs et/ou vibrations, peuvent entraîner une saturation du signal en "faussant" la valeur moyenne du signal de débit de NO (i.e. flux de mélange NO/N₂) mesuré par le capteur de débit 230, ce qui se répercute négativement sur la régulation du débit de NO par contrôle de l'électrovanne proportionnelle 220 par les moyens de pilotage 210, avec risque de fermeture intempestive de l'électrovanne proportionnelle 220 du MFC 240, fournissant le débit de gaz à base de NO, donc d'arrêter prématurément la délivrance de NO aux patients en engendrant alors un effet rebond négatif chez le patient.

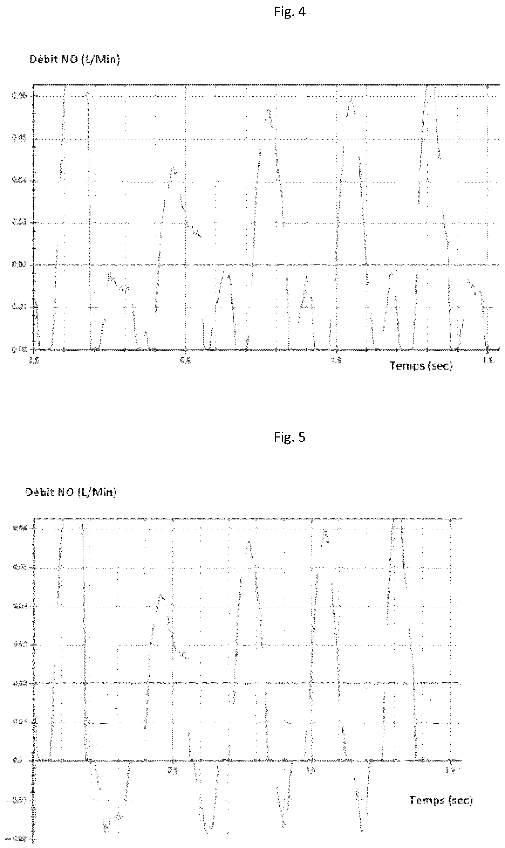

Ce phénomène de saturation du signal en "faussant" la valeur moyenne du signal de débit de NO opéré par un capteur de débit unidirectionnel dit « classique » est illustré par les Fig. 3 et Fig. 4.

Plus précisément, Fig. 3 et Fig. 4 illustrent l'influence des vibrations pouvant se produire lors d'un transport, par exemple en ambulance ou en hélicoptère, d'un patient auquel on fournit du NO gazeux avec un appareil de fourniture de NO 1, tel que décrit ci-avant, lequel est équipé d'un capteur de débit unidirectionnel classique 230 permettant de mesurer les flux de NO que dans un seul sens de circulation du gaz, à savoir en direction du patient (i.e. flèche F en Fig. 2).

Fig. 3 représente la courbe de débit de NO (L/min) régulé pour une consigne constante de débit de NO de 20 mL/min, ce qui est un cas d'usage classique lorsque le NO est fourni au lit du patient, c'est-à-dire à un patient hospitalisé et alité, donc en l'absence de vibrations.

Comme on le voit, la courbe de débit est stable au fil du temps (en sec) et fluctue très peu autour de la valeur de 20 mL/min car l'appareil de fourniture de NO 1 n'est pas en mouvement, donc n'est pas soumis à des vibrations ou analogues. Dans un tel cas d'usage, les mesures de pression opérées par le capteur de pression classique 230, i.e. unidirectionnel, ne sont pas perturbées, du fait de l'absence de toute vibration.

Par ailleurs, Fig. 4 représente, à titre comparatif, la courbe de débit de NO obtenu dans les mêmes conditions (i.e. débit de consigne 20 ml/min) mais lorsque l'appareil de fourniture de NO 1 est soumis à des vibrations, comme celles qu'il peut subir pendant le déplacement du patient, dans l'hôpital, par exemple pour aller réaliser un scanner ou autre, ou de façon encore plus notable, lors d'un transport du patient en véhicule terrestre ou aérien, par exemple durant son transfert d'un hôpital à un autre.

Comme on le voit, dans ce cas, l'amplitude des variations de débit de NO (i.e. les mesures de débit) mesurées par le capteur unidirectionnel classique 230 peuvent dépasser très largement l'amplitude de la consigne de NO, i.e. les fluctuations autour de la valeur de débit de NO fixée à 20 mL/min, montrée en Fig. 3.

Dans ce cas, on assiste alors à une saturation du signal à 0 L/min qui "fausse" la valeur moyenne du signal de débit de NO. Ceci trompe la régulation par les moyens de pilotage 210, ce qui va engendrer une fermeture de l'électrovanne proportionnelle 220 servant au contrôle du débit de NO et arrêter la délivrance de NO au patient, en risquant de produire un effet rebond indésirable chez celui-ci.

Afin de résoudre ce problème, selon l'invention, il est proposé d'utiliser un capteur de débit 230 particulier au sein du circuit 200 afin de pouvoir y mesurer des débits de NO dans les deux sens de circulation du gaz, c'est-à-dire dans le sens de la flèche F et dans le sens opposé, et ce, malgré que l'écoulement du débit gazeux ne se fasse en théorie que dans une direction au sein du circuit de gaz 200, à savoir dans le sens allant vers le patient, c'est-à-dire le sens de la flèche F sur Fig. 2.

Plus précisément, afin de conserver un mode de régulation dit « linéaire », le capteur de débit 230 particulier utilisable dans un appareil de délivrance de NO 1 selon l'invention est un capteur de débit bidirectionnel.

Autrement dit, selon l'invention, un capteur de débit bidirectionnel 230 a été inséré dans le circuit de gaz 200 et testé dans les mêmes conditions que pour la Fig. 4. Fig. 5 représente la courbe de débit de NO obtenue pour un débit de consigne de 20 ml/min et un appareil 1 soumis à des vibrations, avec l'appareil de fourniture de NO 1 embarquant un tel capteur de débit bidirectionnel 230.

On constate que le capteur de débit bidirectionnel 210 a mesuré des flux négatifs correspondant aux portions de courbe situées sous l'axe des abscisses, c'est-à-dire pour les valeurs de débit inférieures à 0.0 mL/min.

Ces débits négatifs étaient considérés, de manière erronée, comme des débits positifs ou nuls lorsque mesurés par le capteur classique utilisé lors de l'essai illustré sur Fig. 4 et conduisaient à un pilotage erroné de l'électrovanne 220 par les moyens de pilotage 210 qui recevaient et traitaient ces mesures de débits erronées.

Or, grâce au capteur de débit bidirectionnel 230 utilisé en lieu et place du capteur unidirectionnel classique, ce phénomène est évité grâce à une prise en compte des flux négatifs engendrés par les vibrations causées lors du transport de l'appareil 1 et du patient P.

En effet, les chocs ou les vibrations engendrent des variations symétriques sur le signal de débit, c'est-à-dire positif et négatif. Dès lors, en prenant en compte les débits négatifs, la valeur moyenne du signal est alors correcte car les alternances positives et négatives engendrées par les vibrations s'annulent.

Ceci permet dès lors aux moyens de pilotage 210 de mieux piloter l'électrovanne proportionnelle 220 en évitant de la fermer lorsque cela n'est pas nécessaire, grâce à une valeur moyenne cohérente.

Une installation d'administration de gaz 100 incluant un appareil de délivrance de NO 1 selon l'invention peut être utilisée pour administrer par inhalation du monoxyde d'azote (NO), i.e. le mélange final obtenu NO/O₂/N₂, aux personnes, i.e. patients, souffrant d'hypertension artérielle pulmonaire aiguë, notamment pour opérer une dilatation de leurs vaisseaux pulmonaires et une augmentation de leur oxygénation en améliorant les échanges gazeux pulmonaires, en particulier pour traiter l'Hypertension Artérielle Pulmonaire du Nouveau-né ou PPHN, le Syndrome de Détresse Respiratoire Aigüe ou SDRA observé principalement chez l'adulte, ou les hypertensions pulmonaires (HP) en chirurgie cardiaque chez l'adulte ou l'enfant.

## Revendications

1. Appareil de délivrance (1) d'un gaz contenant du NO comprenant :
- un circuit de gaz interne (200) pour acheminer un flux de gaz contenant du NO, comprenant des moyens de contrôle de débit (220, 230, 240) configurés pour contrôler le flux de gaz contenant du NO au sein du circuit de gaz (200), lesdits moyens de contrôle de débit (220, 230, 240) comprenant une électrovanne proportionnelle (220) et un dispositif de mesure débit (230), et
- des moyens de pilotage (210) coopérant avec les moyens de contrôle de débit (220, 230, 240) pour autoriser, interdire ou ajuster le débit gazeux au sein dudit circuit de gaz (200),
**caractérisé en ce que** le dispositif de mesure débit (230) comprend un capteur de débit bidirectionnel configuré pour :
- opérer des mesures de débit positives et négatives du flux gazeux contenant du NO circulant dans le circuit de gaz (200), et
- fournir lesdites mesures de débit aux moyens de pilotage (210), lesdits moyens de pilotage (210) traitant les mesures de débit positives et négatives provenant du dispositif de mesure débit (230) pour piloter l'électrovanne proportionnelle (220).

2. Appareil selon la revendication 1, **caractérisé en ce que** les moyens de pilotage (210) sont configurés pour piloter l'électrovanne proportionnelle (220) à partir d'une valeur moyenne de débit prenant en compte les débits négatifs fournis par le capteur de débit bidirectionnel.

3. Appareil selon l'une des revendications 1 ou 2, **caractérisé en ce que** le dispositif de mesure débit (230) est agencé dans le circuit de gaz (200) en amont de l'électrovanne proportionnelle (220).

4. Appareil selon l'une des revendications 1 ou 2, **caractérisé en ce que** les moyens de pilotage (210) comprennent au moins un microprocesseur (211).

5. Appareil selon l'une des revendications 1 à 3, **caractérisé en ce que** l'électrovanne proportionnelle (220) et le dispositif de mesure débit (230) forment tout ou partie d'un contrôleur de débit massique (240)

6. Appareil selon la revendication 1, **caractérisé en ce qu'**il comprend des moyens de réglage de dose de NO configurés pour permettre à un utilisateur de régler une dose de NO désirée.

7. Appareil selon la revendication 6, **caractérisé en ce que** les moyens de réglage de dose de NO sont configurés pour permettre à un utilisateur de régler une dose de NO comprise entre 1 et 80 ppmv.

8. Appareil selon l'une des revendications 1 et 6 ou 7, **caractérisé en ce qu'**il comprend en outre un afficheur graphique (4) et les moyens de pilotage (210) sont configurés pour commander un affichage sur l'afficheur graphique (4) de la dose de NO réglée.

9. Appareil selon les revendications 6 ou 7 et 8, **caractérisé en ce que** les moyens de pilotage (210) sont configurés pour commander un affichage sur l'afficheur graphique (4) de seuils d'alarme NO haute et bas déterminés par les moyens de pilotage (210) en fonction de la dose de NO réglée.

10. Appareil selon la revendication 1, **caractérisé en ce que** les moyens de pilotage (210) sont configurés pour ajuster ou contrôler le débit du flux gazeux contenant du NO au sein du circuit (200) en pilotant l'électrovanne proportionnelle (210).

11. Installation de fourniture d'un gaz contenant du NO (100) comprenant l'appareil de délivrance de NO (1) selon l'une des revendications précédentes alimenté en un mélange gazeux NO/N₂ par au moins une source de mélange NO/N₂ (10), et un ventilateur médical (50) configuré pour fournir un flux de gaz respiratoire contenant de l'O₂.

12. Installation selon la revendication 11, **caractérisée en ce que** ladite au moins une source de mélange NO/N₂ (10) comprend une bouteille de gaz contenant un mélange gazeux NO/N₂ contenant entre 100 et 2000 ppmv de NO, le reste étant de l'azote (N₂).

13. Installation selon la revendication 11, **caractérisée en ce que** l'appareil de délivrance de NO (1) et le ventilateur médical (50) sont raccordés fluidiquement à un circuit respiratoire (20) pour lui fournir le flux de gaz contenant le NO et le flux de gaz contenant de l'oxygène, et ainsi obtenir un mélange gazeux combiné à fournir au patient.

14. Installation selon la revendication 13, **caractérisée en ce qu'**un capteur de débit (25) est agencé dans le circuit respiratoire (20) entre le ventilateur médical (50) et un dispositif d'injection (24) agencé dans le circuit respiratoire (20).

15. Installation selon l'une des revendications 6 ou 7 et 8, **caractérisée en ce que** l'afficheur graphique (4) comprend un écran tactile et les moyens de réglage de dose de NO comprennent une ou des touches virtuelles tactiles, actionnables par l'utilisateur, s'affichant sur l'afficheur graphique (4).
